(19) Europäisches Patentamt
European Patent Office
Office européen des brevets

(11) **EP 1 380 834 B1**

(12) **FASCICULE DE BREVET EUROPEEN**

(45) Date de publication et mention
de la délivrance du brevet:
**27.04.2011 Bulletin 2011/17**

(51) Int Cl.:
*G01N 23/06* (2006.01)   *G01N 23/04* (2006.01)
*A61B 6/00* (2006.01)   *G01V 5/00* (2006.01)

(21) Numéro de dépôt: **03102023.3**

(22) Date de dépôt: **04.07.2003**

(54) **Procédé d'examen radiologique d'objets**

Verfahren zur radiologischen Untersuchung von Gegenständen

Method of radiological examination of objects

(84) Etats contractants désignés:
**DE GB IT**

(30) Priorité: **05.07.2002 FR 0208513**

(43) Date de publication de la demande:
**14.01.2004 Bulletin 2004/03**

(73) Titulaire: **Commissariat à l'Énergie Atomique et aux Énergies Alternatives**
**75015 Paris (FR)**

(72) Inventeurs:
• **Herve, Lionel**
  **26500 BOURG-LES-VALENCE (FR)**
• **Robert-Coutant, Christine**
  **38410, Saint-Martin-d'Uriage (FR)**

(74) Mandataire: **Poulin, Gérard et al**
**BREVALEX**
**95 rue d'Amsterdam**
**75378 Paris Cedex 8 (FR)**

(56) Documents cités:
**US-A- 3 965 358**

• **SWANPALMER J ET AL: "MEASUREMENT OF BONE MINERAL USING MULTIPLE-ENERGY X-RAY ABSORPTIOMETRY" PHYSICS IN MEDICINE AND BIOLOGY, TAYLOR AND FRANCIS LTD. LONDON, GB, vol. 43, no. 2, 1 février 1998 (1998-02-01), pages 379-387, XP000732770 ISSN: 0031-9155**
• **ROBERT-COUTANT C ET AL: "Estimation of the matrix attenuation in heterogeneous radioactive waste drums using dual-energy computed tomography" NUCLEAR INSTRUMENTS & METHODS IN PHYSICS RESEARCH, SECTION - A: ACCELERATORS, SPECTROMETERS, DETECTORS AND ASSOCIATED EQUIPMENT, NORTH-HOLLAND PUBLISHING COMPANY. AMSTERDAM, NL, vol. 422, no. 1-3, 11 février 1999 (1999-02-11), pages 949-956, XP004161971 ISSN: 0168-9002**

**Description**

Cette invention concerne un procédé d'examen radiologique multi-énergie d'un objet.

Les procédés radiologiques consistent à faire traverser un objet à étudier par un rayonnement pour en déduire la répartition de différentes catégories de matériaux, absorbant le rayonnement de façons différentes, dans cet objet. Une application très courante est l'ostéodensitométrie, où on recherche la masse et la densité des tissus osseux dans un patient en distinguant ces tissus des tissus mous.

Il est usuel d'utiliser un spectre de rayonnement large et à le diviser en bandes mesurées séparément par des canaux de mesure respectifs. Comme les coefficients d'absorption ou d'atténuation du rayonnement par l'une ou l'autre des catégories de tissu sont différents pour chacune des bandes, le problème théorique se ramène à la solution d'un système d'équations dont le nombre est égal à celui des bandes de mesure et qui comprennent chacune deux inconnues (les épaisseurs ou les masses traversées de tissus mous et osseux). La solution du problème devient possible après une calibration obtenue en faisant traverser par le rayonnement divers étalons dotés d'épaisseurs connues de matériaux aux propriétés d'absorption analogues à celles des matériaux de mesure, et notamment le plexiglas et l'hydroxyapatite pour simuler les tissus mous et les tissus osseux. On peut alors, calculer les paramètres mathématiques d'un modèle reliant les mesures d'atténuation aux épaisseurs des matériaux.

On notera qu'en réalité le corps humain contient trois catégories principales de tissus : les tissus osseux, les tissus maigres et les tissus gras, mais qu'on n'en considère que deux en général à cause de difficultés à distinguer ces trois catégories dans les mesures, de sorte qu'on confond volontairement les tissus maigres et gras. D'autres procédés sont ensuite appliqués pour distinguer leurs proportions dans les tissus mous.

La largeur du spectre permet de disposer d'un nombre beaucoup plus grand de bandes de mesures qu'il ne serait nécessaire pour obtenir une solution, et de les employer toutes pour obtenir des résultats plus précis en exploitant toute l'information d'absorption obtenue. Dans l'article "Measurement of bone mineral using a multiple-energy x-ray absorptiometry", par J. Swanpalmer, R. Kullenberg, T. Hansson, Phys.Med.Biol, Vol 43, 1998: pp 379-387, où on dispose de 23 bandes de mesures et où on considère les trois catégories de tissus, il est proposé de combiner les groupes de mesures trois par trois de toutes les façons possibles pour obtenir 1771 (23x22x21/2x3) systèmes de trois équations à trois inconnues qui donnent autant de groupes de résultats. Il convient alors d'appliquer un critère de choix. Les auteurs conseillent de choisir comme résultat véridique celui qui est à la valeur moyenne, ou à la valeur médiane, pour le paramètre le plus important, qui peut être la masse osseuse traversée.

Contrairement à ce qu'on a conseillé plus haut, les auteurs considèrent les trois catégories de tissus. Cela ne met pas en question la validité de leur méthode, à condition d'exposer le patient à une intensité d'irradiation beaucoup plus forte pour ramener les incertitudes des mesures à des proportions acceptables. Il serait d'ailleurs possible de modifier simplement leur méthode pour l'appliquer à des mesures sur deux tissus, ou plus généralement deux catégories de matériaux, seulement. Toutefois, il subsiste le défaut que cette méthode ne tient pas compte du bruit sur les mesures et qu'elle conduit donc à des résultats bruités.

L'invention a pour objet un perfectionnement de tels procédés par combinaison de résultats nombreux et comprend une amélioration du critère de choix des résultats. Plus précisément, elle concerne sous sa forme la plus générale un procédé d'examen radiologique d'un objet où sont considérées au moins deux catégories de matériaux selon la revendication 1. L'application d'un critère de choix parmi les expressions ($\hat{M}$) pour en déduire une expression ($\hat{M}$ finale) jugée véridique est caractérisée en ce que le critère de choix comprend un combinaison (f) des expressions avec des facteurs de pondération (a), et un calcul de facteurs de pondération de manière que la combinaison ait un bruit minimal (variance minimale en langage mathématique) calculé d'après les bruits sur les mesures (variances sur les mesures).

L'invention sera maintenant décrite en référence aux figures :

- la figure 1 est une vue d'un spectre,
- la figure 2 est une répartition de résultats,

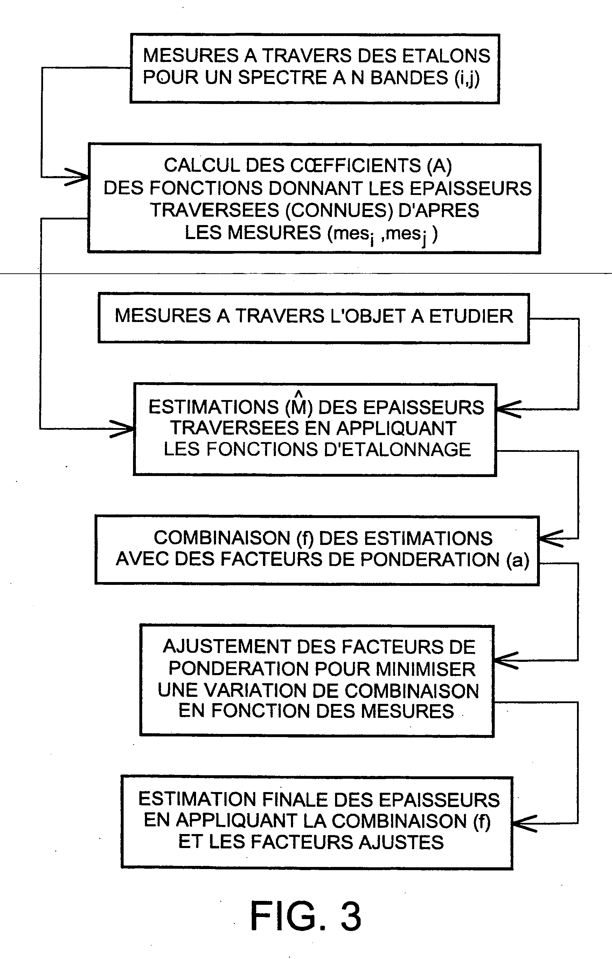

- et la figure 3 est un organigramme résumant l'invention, auquel on pourra se reporter dans toute la description qui suit.

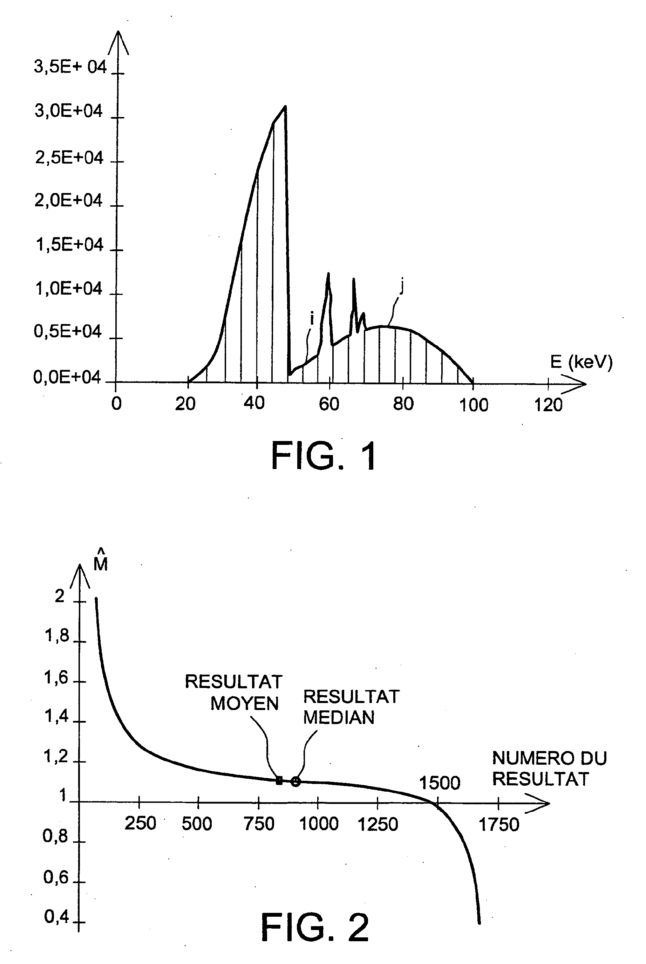

L'atténuation du rayonnement peut être exprimée par une fonction des épaisseurs traversées de chacun des matériaux d'indices x et y, ou de leurs masses M (densités par unité de surface) dans la direction du rayonnement. Le spectre de mesures de la figure 1 est divisé en N bandes notées généralement par les indices i et j. Les atténuations varieront dans chacune des bandes en raison de coefficients d'absorption variables pour les deux matériaux. Si nous appelons $mes_i$ ou $mes_j$ les mesures pour une bande d'énergie i ou j, les masses traversées $M_x$ et $M_y$ pourront chacune être exprimées aussi, par exemple, par la formule générale $\hat{M}$ $=A_1+A_2.mes_i+A_3.mes_j+A_4.mes^2_i+A_5.mes^2_j+A_6.mes_i.mes_j$. Les mesures considérées dans cet exemple étant des meusures d'atténuation, on aura pour chaque canal de mesure i (correspondant à une bande du spectre) la relation $mes_i = \ln(^{noi}/_{ni})$ où noi est le nombre de photons arrivant sur l'objet et ni celui des photons ayant traversé l'objet. Comme la non-linéarité des fonctions $M_x$ et $M_y$ en fonction des mesures est faible en pratique, on peut s'accommoder de cette fonction polynomiale du deuxième degré qui comprend six coefficients $A_1$ à $A_6$.

Le degré du polynôme peut être ajusté en fonction du problème. Par exemple, pour l'analyse d'objets constitués de matériaux ayant des numéros atomiques plus élevés que celui des tissus biologiques, comme le contrôle non destructif pour l'examen d'un objet métallique.

Ces coefficients peuvent être trouvés dans une étape d'étalonnage à travers des étalons, appelés parfois fantômes ou cales dans l'art, et qui consistent en des pièces formées d'épaisseurs connues et différentes entre elles de matériaux simulant, par leurs propriétés d'atténuation, les matériaux de l'objet qui sera effectivement à mesurer. Chacun des étalons est donc soumis à une irradiation par le rayonnement pendant une longue durée qui permet de réduire l'influence du bruit sur les mesures. Le spectre mesuré pour chacun des étalons donne encore N mesures résultant de la décomposition du spectre en autant de bandes. En combinant maintenant deux séries de mesures i et j prises pour deux bandes et pour chacun des étalons, on recherche les coefficients A pour ajuster les fonctions $M_x$ et $M_y$ aux mesures. Dans le cas présent, où il y a six coefficients A pour chacune des deux fonctions, et où on considère les combinaisons de deux bandes de mesure, les mesures pour chacune des bandes devront, pour fournir une solution unique, porter sur six étalons. Des étalons plus nombreux pourront aussi être utilisés pour améliorer la précision sur la solution. Une minimisation de fonction d'erreur sera alors appliquée.

Cette détermination des coefficients A est répétée pour diverses combinaisons de paires de mesures. Il en était encore ainsi dans l'article antérieur mentionné plus haut ; cependant, on a constaté qu'il était inutile d'effectuer toutes les combinaisons, au nombre de

$$\frac{N \times (N-1)}{2},$$ , pour exploiter complètement les mesures et que (N-1) combinaison étaient en réalité suffisantes pour recueillir toute l'information.

Une façon préférée de procéder consiste à choisir au départ la bande de mesures qui est la moins bruitée (par exemple celle qui a le signal n- le nombre de photons à la réception- le plus important) et à l'associer successivement à chacune des autres bandes de mesures pour les combinaisons. 0n obtient finalement (N-1) estimations des deux fonctions $M_x$ et $M_y$, qu'on note $\hat{M}_1, \hat{M}_2,..., \hat{M}_{N-1}$ pour chacune de ces deux fonctions.

A ce stade du procédé, les fonctions $M_x$ et $M_Y$, représentatives des longueurs traversées de deux matériaux représentatifs des tissus osseux et des tissus mous, pourront être converties en fonction $M_U$, $M_v$ et $M_w$ représentatives des longueurs équivalentes traversées des tissus osseux, des tissus maigres et des tissus gras en combinant linéairement $M_x$ et $M_y$ de trois façons différentes déterminées par l'expérience. Ce procédé de conversion est indépendant de l'invention, déjà connu dans l'art.

Comme il n'y a pas de raison de préférer l'une ou l'autre de ces estimations $\hat{M}$, un critère de choix doit être appliqué pour obtenir l'estimation $\hat{M}$ finale qui sera jugée véridique. Dans l'article antérieur, une des expressions obtenues était directement sélectionnée d'après un critère de classement (le résultat médian) ou de moyenne des valeurs prises par les expressions pour un des résultats. Dans l'invention, les expressions $\hat{M}$ seront combinées, par exemple linéairement, d'après la formule $\hat{M}$ finale = $(a_1 \hat{M}_1)+(a_2 \hat{M}_2) +...+ (a_{N-1} \hat{M}_{N-1})$ tout en minimisant le bruit ; les coefficients $a_1$, etc. ont une somme égale à l'unité $(a_1 + a_2 + ....+ a_{N-1} = 1)$.

Pour chaque canal de mesure, le bruit sur le nombre de photons suit une loi statistique de Poisson, dont le résultat est indépendant pour chacun des canaux. La matrice de covariance des N-1 résultats peut être exprimée d'après la formule

$$\Gamma_{ij} = \sum_{k=1}^{N-1} \frac{\partial \hat{M}_i}{\partial mes_k} \frac{\partial \hat{M}_j}{\partial mes_k} \frac{1}{N_k} \, .$$

La variance sur la combinaison linéaire donnant $\hat{M}$ finale s'exprime par la formule f =$(a_1,...,a_{N}-1) \cdot \Gamma \cdot {}^t (a_1,..., a_{N-1})$ ; cette quantité f atteint une valeur optimale quand sa dérivée selon toutes les variables est nulle, c'est-à-dire que l'influence du bruit est minimisée, soit :

$$\begin{cases} \partial f / \partial a_1 = 0 \\ \partial f / \partial a_2 = 0 \\ ... \\ \partial f / \partial a_{N-1} = 0 \end{cases}$$

La résolution numérique de ce système fournit les coefficients $a_1$, $a_2$, etc et $\hat{M}$ finale, c'est-à-dire les masses traversées des deux catégories de matériaux.

Ce sont ces opérations qui sont accomplies dans l'invention ; il est important de remarquer que les mesures ($mes_k$) employées dans la formule donnant $\Gamma_{ij}$ et ensuite sont les mesures effectuées à travers l'objet à étudier lui-même, mais pas celles qui ont été faites à l'étalonnage pour déterminer les coefficients A des fonctions $\hat{M}$.

L'invention permet d'échapper au dilemme de procédés antérieurs dans lesquels on renonçait à une partie des énergies de mesure ou au contraire on acceptait des mesures peu distinctes : elle utilise en effet tout le spectre, mais en le divisant en bandes assez nombreuses pour que la mesure de chacune puisse être comparée de façon utile à d'autres mesures, faites sur des bandes éloignées. Elle permet ainsi, entre autres avantages, d'étudier aussi bien des organismes maigres que gras.

Enfin, l'invention peut être généralisée à un nombre de matériaux plus grand que deux, ce qui peut être intéressant notamment pour les procédés d'imagerie à produit de contraste, où trois variables doivent être considérées ou pour contrôle des bagages (recherche d'explosifs) ; et elle peut être appliquée en considérant des combinaisons autres que linéaires des fonctions $\hat{M}$.

Des application de l'invention sont :

. l'ostéodensitométrie

- obtention de la densité de masse osseuse
- obtention de la composition corporelle (masse grasse, masse maigre)

. le contrôle agro-alimentaire, par exemple la détection de fragments d'os dans de la viande ou la détection de morceaux de verres dans des plats cuisinés,

. le contrôle des bagages : recherche d'explosifs, de produits illicites (armes, aliments, drogue...)

## Revendications

1. Procédé d'examen radiologique d'un objet où sont considérées au moins deux catégories de matériaux, comprenant : l'emploi d'un rayonnement à spectre large pour effectuer des mesures ($mes_k$) d'atténuation du rayonnement par N bandes du spectre ; l'emploi d'au moins N-1 expressions ($\hat{M}$) d'épaisseurs ou de masses pour chacune des catégories de matériaux traversées par le rayonnement, les expressions ($\hat{M}$) étant des fonctions des diverses combinaisons de paires de mesures ($mes_k$) et de coefficients (A) déterminés par étalonnage ; et l'application d'un critère de choix parmi les plusieurs expressions ($\hat{M}$) pour en déduire une expression ($\hat{M}$ finale) jugée véridique ; **caractérisé en ce que** le critère de choix comprend une combinaison (f) des expressions ($\hat{M}$) avec des facteurs de pondération (a), et un calcul des facteurs de pondération de manière que la combinaison ait un bruit minimal calculé d'après la variance sur le nombre de photons des mesures.

2. Procédé selon la revendication 1, **caractérisé en ce que** la combinaison (f) des expressions est linéaire.

3. Procédé selon l'une quelconque des revendications 1 ou 2, **caractérisé en ce que** la variance de la combinaison est calculée avec une matrice de covariance ($\Gamma_{ij}$) des expressions ($\hat{M}$).

4. Procédé selon l'une quelconque des revendications 1 à 3, **caractérisé en ce que** les expressions ($\hat{M}$)

sont en nombre égal à celui des bandes, moins un, et établies toujours avec une des bandes (io) et, respectivement, chacune des autres bandes.

5. Procédé selon l'une quelconque des revendications 1 à 4, **caractérisé en ce que** les coefficients (A) sont déterminés à une étape préalable de calibration.

6. Procédé selon l'une quelconque des revendications précédente, **caractérisé en ce qu'**il est appliqué à l'ostéodensitométrie.

7. Procédé selon l'une quelconque des revendications 1 à 5, **caractérisé en ce qu'**il est appliqué à des contrôles agro-alimentaires.

8. Procédé selon l'une quelconque des revendications 1 à 5, **caractérisé en ce qu'**il est appliqué au contrôle de bagages.

## Claims

1. Method for x-ray examination of an object where at least two categories of materials are taken into consideration, comprising: the use of broad spectrum x-rays; measurements of the x-rays by bands of the spectrum; expressions ($\hat{M}$) of thicknesses or masses of the two categories of materials passed through by the x-rays, the expressions ($\hat{M}$) being functions of at least two of the measurements ($mes_k$) and coefficients (A) : and applying a selection criterion from among the expressions ($\hat{M}$) to deduce from this an expression (final $\hat{M}$) considered true; **characterized in that** the selection criterion comprises a combination (f) of the expressions with weighting factors (a), and a calculation of the weighting factors such that the combination has minimal noise calculated according to a noise level on the measurements.

2. Method according to claim 1, **characterized in that** the combination (f) of the expressions is linear.

3. Method according to claim 1, **characterized in that** the variation of the combination is calculated with a covariance matrix ($\Gamma_{ij}$) of the ($\hat{M}$) expressions.

4. Method according to claim 1, **characterized in that** there are as many ($\hat{M}$) expressions as there are bands minus one, and the expressions are also established with one of the bands (io) and each of the other bands, respectively.

5. Method according to claim 1, **characterized in that** the coefficients (A) are determined in a preliminary calibration step.

**6.** Method according to claim 1, **characterized in that** it is applied to osteodensitometry.

**7.** Method according to claim 1, **characterized in that** it is applied to food-processing inspections.

**8.** Method according to claim 1, **characterized in that** it is applied to baggage inspection.

**Patentansprüche**

**1.** Verfahren zur radiologischen Untersuchung eines Gegenstands, bei dem wenigstens zwei Kategorien von Materialien in Betracht gezogen werden, umfassend: die Anwendung einer Strahlung mit breitem Spektrum, um Strahlungsdämpfungsmessungen ($mes_k$) mit N Spektrumsbändern durchzuführen; die Anwendung von wenigstens N-1 Dicken- oder Massenausdrücken ($\hat{M}$) für jede der durch die Strahlung durchquerten Materialkategorien, wobei die Ausdrücke ($\hat{M}$) Funktionen der diversen Kombinationen von Messungspaaren ($mes_k$) und von durch Kalibrierung bestimmten Koeffizienten (A) sind; und die Anwendung eines Auswahlkriteriums zwischen mehreren Ausdrücken ($\hat{M}$), um davon einen als wahr bewerteten Ausdruck ($\hat{M}$ final) abzuleiten; **dadurch gekennzeichnet, dass** das Auswahlkriterium eine Kombination (f) der Ausdrücke ($\hat{M}$) mit Gewichtungsfaktoren (a) und eine Berechung der Gewichtungsfaktoren in der Art umfasst, dass die Kombination ein gemäß der Varianz bei Messungen berechnetes minimales Geräusch aufweist.

**2.** Verfahren nach Anspruch 1, **dadurch gekennzeichnet, dass** die Kombination (f) der Ausdrücke linear ist.

**3.** Verfahren nach einem der Ansprüche 1 oder 2, **dadurch gekennzeichnet, dass** die Varianz der Kombination (f) mit einer Covarianz-Matrix $\Gamma_{ij}$ der Ausdrücke ($\hat{M}$) berechnet wird.

**4.** Verfahren nach einem der Ansprüche 1 bis 3, **dadurch gekennzeichnet, dass** die Anzahl der Ausdrücke ($\hat{M}$) gleich derjenigen der Bänder minus 1 ist und immer mit einem der Bänder (io) und, beziehungsweise, jedem der anderen Bänder ermittelt wird.

**5.** Verfahren nach einem der Ansprüche 1 bis 4, **dadurch gekennzeichnet, dass** die Koeffizienten (A) in einem vorausgehenden Kalibrierungsschritt bestimmt werden.

**6.** Verfahren nach einem der vorhergehenden Ansprüche, **dadurch gekennzeichnet, dass** es bei der Osteodensitometrie angewendet wird.

**7.** Verfahren nach einem der Ansprüche 1 bis 5, **dadurch gekennzeichnet, dass** es bei Lebensmittelkontrollen angewendet wird.

**8.** Verfahren nach einem der Ansprüche 1 bis 5, **dadurch gekennzeichnet, dass** es bei der Gepäckkontrolle angewendet wird.

FIG. 1

FIG. 2

MESURES A TRAVERS DES ETALONS
POUR UN SPECTRE A N BANDES (i,j)

CALCUL DES CŒFFICIENTS (A)
DES FONCTIONS DONNANT LES EPAISSEURS
TRAVERSEES (CONNUES) D'APRES
LES MESURES (mes$_i$ ,mes$_j$ )

MESURES A TRAVERS L'OBJET A ETUDIER

ESTIMATIONS ($\hat{M}$) DES EPAISSEURS
TRAVERSEES EN APPLIQUANT
LES FONCTIONS D'ETALONNAGE

COMBINAISON (f) DES ESTIMATIONS
AVEC DES FACTEURS DE PONDERATION (a)

AJUSTEMENT DES FACTEURS DE
PONDERATION POUR MINIMISER
UNE VARIATION DE COMBINAISON
EN FONCTION DES MESURES

ESTIMATION FINALE DES EPAISSEURS
EN APPLIQUANT LA COMBINAISON (f)
ET LES FACTEURS AJUSTES

# FIG. 3

# EP 1 380 834 B1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **J. Swanpalmer ; R. Kullenberg ; T. Hansson.** Measurement of bone mineral using a multiple-energy x-ray absorptiometry. *Phys.Med.Biol,* 1998, vol. 43, 379-387